# EUROPEAN PATENT APPLICATION

(11) **EP 4 582 141 A2**
(43) Date of publication of application: **09.07.2025**
(21) Application number: 25178056.5
(22) Date of filing: 21.11.2017
(51) Int. Cl.: A61P 1/04

(54) **ORAL COMPOSITION FOR THE TREATMENT OF GASTROESOPHAGEAL REFLUX AND LARYNGOPHARYNGEAL REFLUX**

(30) Priority: 30.11.2016 IT 201600121601
(62) Divisional of application: 17817880.2
(71) Applicant: Nekkar Lab S.r.l., 20131 Milano (IT)
(72) Inventor: DI TRAPANI, Nicola, Milano (IT); PALLADINI, Francesco, Milano (IT)
(74) Representative: Villa, Livia

(57) **Abstract**

The present invention relates to a composition comprising at least one salt of alginic acid, hyaluronic acid, a natural molecule, and thickening agents. Said composition has been demonstrated to effectively counteract the discomfort caused by gastroesophageal reflux, preventing said reflux and alleviating the effects thereof on the mucosa. Indeed, the composition can also play an active role in the regeneration of tissue damaged by previous reflux.

## Description

### FIELD OF INVENTION

The present invention relates to a composition comprising at least one salt of alginic acid, hyaluronic acid, a natural molecule, and thickening agents. Said composition has been demonstrated to effectively counteract the discomfort caused by gastroesophageal reflux and laryngopharyngeal reflux, preventing said reflux and alleviating the effects thereof on the mucosa. Indeed, the composition can also play an active role in the regeneration of tissue damaged by previous reflux.

### STATE OF THE ART

The exponential increase in the number of patients with symptoms relating to gastroesophageal motility disorders and gastroesophageal reflux disorders is now widely recognized by the medical and scientific community. The anatomical and functional factors are multiple and the causes that trigger or maintain reflux diseases and/or the expressions thereof are likewise multifactorial.

In addition, further factors include the ineffectiveness of the treatments currently in use and, above all, the abuse of drugs which do not act on the pathophysiological mechanisms of the diseases in question.

It is also important to acknowledge the seriousness of the consequences of these diseases for the gastric mucosa and above all for the esophageal mucosa, where acute and chronic lesions occur. Indeed, the constant presence of wounding stimuli makes healing somewhat difficult; furthermore, the healing process is carried out by means of scar formation, rather than by tissue regeneration, and the constant presence of said scars alters gastroesophageal function.

In addition to these considerations, there is also an increasing number of diseases affecting the (upper and lower) airways attributable and ascribable to the acid reflux or gastric vapour mechanisms.

The object of the present invention is therefore to offer a practical and effective remedy, which is also well tolerated by patients, for the treatment of gastroesophageal reflux and disorders associated therewith.

### SUMMARY OF INVENTION

Said object has been achieved by a composition as described in claim 1.

In another aspect, the present invention relates to a process for preparing the composition. In another aspect, the present invention relates to the use of said composition in the treatment of gastroesophageal reflux and laryngopharyngeal reflux.

As will be evident from the following detailed description and the embodiments provided as non-limiting examples, the composition according to the invention has surprisingly shown reparative and regenerative properties with respect to said mucosa, in addition to mucoadhesive and filmogenic characteristics which increase contact time with said mucosa and, consequently, effectiveness.

### DETAILED DESCRIPTION OF THE INVENTION

Therefore, the invention relates to a composition comprising:
i) at least one salt of alginic acid, and
ii) an active component comprising:
   - hyaluronic acid or a salt thereof, said salt being selected from sodium hyaluronate, potassium hyaluronate, calcium hyaluronate, and mixtures thereof,
   - a natural molecule selected from glycosaminoglycan, glycoprotein, keratin, collagen, and mixtures thereof, and
   - at least one thickening agent,
wherein said at least one salt of alginic acid and active component are in a weight ratio of 1:1 to 1:15.

Indeed, it has been surprisingly found that the composition of the invention offers advantageous reparative and regenerative properties with respect to the mucosa, with consequent rapid relief from symptoms, featuring, at the same time, significant rheological characteristics both in terms of transit and in terms of mucoadhesive properties of the components thereof, with a direct causality between contact time and efficacy.

The composition of the invention therefore benefits from the association of the salt of alginic acid and the active component, i.e. from the mucoadhesive, regenerating and filmogenic characteristics, as well as from the important conveyance of water, hyaluronic acid or the salt thereof, strengthened by the presence of the natural molecule, in synergy with the salt of alginic acid.

The efficacy of the association is enhanced and optimised by the contemporaneous presence of at least one thickening agent, which has proper affinity with the proteins of the gastroesophageal mucosa. This allows the composition of the invention to form a film on the surface of the mucosa, thus increasing contact times and therefore supporting the protective and regenerative effect.

The presence of at least one salt of alginic acid maximises the effects of the active component. The salt of alginic acid reacts with the gastric acids, releasing alginic acid, which gels in the presence of water. The resulting gel tends to obstruct reflux of the contents of the stomach into the oesophagus. Indeed, said gel is a floating gel which forms what is known as a "raft" on the surface of the gastric contents, i.e. a "stopper" at the lower oesophageal sphincter level, which acts as a kind of neutral insulator that occupies the oesophageal space when the pressure wave pushes the gastric contents towards the oesophagus. The result is that gastric material is physically prevented from back-flowing into the oesophagus.

In the weight ratios stated, it has been advantageously observed that the salt of alginic acid maintains such properties, without altering the stability, solubility, and rheological properties of the composition. Therefore, one observes a synergy between the action of the active component and the action of the salt of alginic acid, since the latter shields the gastric reflux, while also offering the advantage of longer contact time between the active component and the mucosa to be regenerated.

Said salt of alginic acid is preferably sodium alginate, potassium alginate, calcium alginate, magnesium alginate, ammonium alginate, propylene glycol alginate, or a mixture thereof.

In preferred embodiments, said salt of alginic acid is magnesium alginate.

Preferably, said active component is present in an amount greater than said at least one salt of alginic acid. More preferably, said at least one salt of alginic acid and active component are in a weight ratio of 1:2 to 1: 10, and even more preferably of 1:3 to 1:8. In certain embodiments, the composition of the invention has a pH of 6-8, preferably 6.5-7.5, once dissolved in water.

In other preferred embodiments, the composition of the invention further comprises water and has a pH of 6-8. More preferably, the composition of the invention further comprises water and has a pH of 6.5-7.5.

Preferably, said at least one salt of alginic acid is in a concentration of 0.1-1.0 wt% more preferably 0.2-0.5 wt%, on the weight of the composition.

Preferably, said active component is in a concentration of 0.1-10 wt%, more preferably 0.2-5 wt%, on the weight of the composition.

Preferably, said hyaluronic acid or a salt thereof has a molecular weight of 100 kDa-1,500 kDa.

Preferably, the composition of the invention comprises sodium hyaluronate.

Said natural molecule is selected from glycosaminoglycan, glycoprotein, keratin, collagen, and mixtures thereof.

'Glycosaminoglycan' is understood as meaning chondroitin sulphate, dermatan sulphate, keratan sulphate, heparin, heparan sulphate or a mixture thereof.

'Glycoprotein' is understood as meaning mucoprotein, integrin, fibronectin, laminin, vitronectin, thrombospondin, tenascin, entactin, nephronectin, fibrinogen, undulin, or a mixture thereof.

It should be appreciated that the natural molecule in the composition of the present invention is natural because such molecule consists of components which are found naturally in the extracellular matrix and therefore offers complete biocompatibility in addition to playing an active role in hydrating and regenerating damaged tissues.

Said natural molecule preferably is keratin, and more preferably hydrolysed keratin. Indeed, hydrolysed keratin noticeably increases the regeneration process of desmosomes, of which it is a fundamental component, providing a significant boost to the regeneration process in the event of any lesions of the mucosa triggered by hyaluronic acid or salt thereof and acting directly on the extracellular matrix.

Preferably, in the active component, said hyaluronic acid or salt thereof and natural molecule are in a weight ratio of 3:1 to 1:3.

In preferred embodiments, said hyaluronic acid or salt thereof and natural molecule are in a weight ratio of 2:1 to 1:2. Particularly preferable are the compositions in which said hyaluronic acid or salt thereof and natural molecule are in a weight ratio of about 1:1.

In further preferred embodiments, said at least one thickening agent and hyaluronic acid or salt thereof are in a weight ratio of 4:1 to 12:1. More preferably, said at least one thickening agent and hyaluronic acid or salt thereof are in a weight ratio of 6:1 to 10:1. Particularly preferable are the compositions in which said at least one thickening agent and hyaluronic acid or salt thereof are in a weight ratio of 8:1 to 9:1.

In further preferred embodiments, said at least one thickening agent and natural molecule are in a weight ratio of 4:1 to 12:1. More preferably, said at least one thickening agent and natural molecule are in a weight ratio of 6:1 to 10:1. Particularly preferable are the compositions in which said at least one thickening agent and natural molecule are in a weight ratio of 8:1 to 9:1.

Preferably, said hyaluronic acid or salt thereof is in a concentration of up to 0.5 wt% based on the active component weight, and more preferably up to 0.3 wt%. Particularly preferable are compositions in which said hyaluronic acid or salt thereof is in a concentration of 0.05-0.2 wt% based on the active component weight.

Preferably, said natural molecule is in a concentration of up to 0.5 wt% based on the active component weight, and more preferably up to 0.3 wt%. Particularly preferable are compositions in which said natural molecule is in a concentration of 0.05-0.2 wt% based on the active component weight.

Preferably, said at least one thickening agent is in a concentration of up to 5 wt% based on the active component weight, and more preferably up to 2.5 wt%. Particularly preferable are compositions in which said at least one thickening agent is in a concentration of 0.5-1.5 wt% based on the active component weight.

Preferably, said at least one thickening agent is xanthan gum, guar gum, tara gum, locust bean gum, fenugreek gum, arabic gum, calcium carboxymethylcellulose, sodium carboxymethylcellulose, ethyl cellulose, gelatin, ethyl cellulose, hydroxyethyl cellulose, hydroxypropyl cellulose, polydextrose, carrageenan, methylcellulose, sucrose, sorbitol, xylitol, dextrose, fructose, maltitol, tragacanth gum, pectin, agar-agar, carboxypolymethylene, hydroxypropyl methylcellulose, or a mixture thereof.

More preferably, said at least one thickening agent is xanthan gum, guar gum, tara gum, locust bean gum, fenugreek gum, arabic gum, tragacanth gum, carrageenan or a mixture thereof. Said agents are natural gums, which, due to the chemical characteristics thereof, have a high affinity with the proteins of the gastroesophageal mucosa.

In preferred embodiments, the composition of the invention comprises a mixture of xanthan gum and tara gum. Indeed, it has been observed that this mixture offers an optimal combination of advantageous aspects, including adequate oesophageal transit time, which is actually comparable to that of a simple food bolus, adequate formation of film on the mucosa, and, contemporaneously, easy swallowability by the patient.

Preferably, the composition of the invention comprises:
- 0.1-1.0 wt% of at least one salt of alginic acid,
- 0.1-10 wt% of active component, and
- water,
on the weight of the composition.

More preferably, the composition of the invention comprises:
- 0.2-0.5% wt% of at least one salt of alginic acid,
- 0.2-5 wt% of active component, and
- water,
on the weight of the composition.

Preferably, said active component comprises:
- up to 0.5 wt% of hyaluronic acid or salt thereof,
- up to 0.5 wt% of natural molecule, and
- up to 5 wt% of at least one thickening agent,
on the active component.

More preferably, said active component comprises:
- up to 0.3 wt% of hyaluronic acid or salt thereof,
- up to 0.3 wt% of natural molecule, and
- up to 2.5 wt% of at least one thickening agent,
on the weight of the active component.

It should be understood that all the possible combinations of the preferred aspects of the components of the composition are described herein and therefore are also preferred. The composition of the invention may also comprise pharmaceutically acceptable excipients, such as antioxidants, tonicity regulators, preservatives, flavourings, sweeteners, thinners, glidants, colourings, binders, adsorbents, release retardants, and mixtures thereof.

Suitable excipients may be potassium sorbate, sodium benzoate, ε-polylysine, sucralose, maltodextrin, magnesium carbonate, magnesium stearate, natural starch, partially hydrolysed starch, lactose, calcium phosphate, calcium carbonate, calcium sulphate, polyvinylpyrrolidone, silica, colloidal silica, precipitated silica, magnesium silicates, aluminium silicates, sodium lauryl sulphate, magnesium lauryl sulphate, methacrylate copolymers, sodium dehydroacetate, and mixtures thereof.

In certain embodiments, the composition of the invention consists essentially of (i) at least one salt of alginic acid, and (ii) one active component comprising:
- hyaluronic acid or a salt thereof, said salt being selected from sodium hyaluronate, potassium hyaluronate, calcium hyaluronate, and mixtures thereof,
- a natural molecule selected from glycosaminoglycan, glycoprotein, keratin, collagen, and mixtures thereof, and
- at least one thickening agent,
wherein said at least one salt of alginic acid and active component are in a weight ratio of 1:1 to 1: 15. The expression " consists essentially of" means that at least one salt of alginic acid, hyaluronic acid or salt thereof and natural molecule are the only active ingredients for treatment of the gastroesophageal reflux present in the composition, while any other components or excipients do not interfere with the action thereof, and are water-miscible and water-soluble.

In further embodiments, the composition of the invention consists of water (i) at least one salt of alginic acid, and (ii) one active component comprising:
- hyaluronic acid or a salt thereof, said salt being selected from sodium hyaluronate, potassium hyaluronate, calcium hyaluronate, and mixtures thereof,
- a natural molecule selected from glycosaminoglycan, glycoprotein, keratin, collagen, and mixtures thereof, and
- at least one thickening agent,
wherein said at least one salt of alginic acid and active component are in a weight ratio of 1:1 to 1:15.

The composition of the invention can be in the form of a unit dose.

Preferably, said unit dose comprises:
- up to 200 mg of at least one salt of alginic acid,
- up to 3000 mg of active component, and
- up to 100 ml of water.

More preferably, said unit dose comprises:
- up to 100 mg of at least one salt of alginic acid,
- up to 1500 mg of active component, and
- up to 50 ml of water.

In unit dose embodiments, the active component comprises:
- up to 50 mg of hyaluronic acid or salt thereof,
- up to 50 mg of natural molecule, and
- up to 500 mg of at least one thickening agent.

Preferably, the active component comprises:
- up to 30 mg of hyaluronic acid or salt thereof,
- up to 30 mg of natural molecule, and
- up to 250 mg of at least one thickening agent.

In preferred embodiments, said unit dose comprises:
- 20-50 mg of at least one salt of alginic acid,
- 10-20 mg of hyaluronic acid or salt thereof,
- 10-20 mg of natural molecule,
- 100-200 mg of at least one thickening agent, and
- 1-30 ml of water.

In more preferable embodiments, said unit dose comprises:
- 20-50 mg of at least one salt of alginic acid,
- 10-20 mg of hyaluronic acid or salt thereof,
- 10-20 mg of natural molecule,
- 100-200 mg of a mixture of xanthan gum and tara gum, and
- 5-20 ml of water.

Particularly preferable are unit doses comprising:
- 20-50 mg magnesium alginate,
- 10-20 mg of sodium hyaluronate,
- 10-20 mg of natural molecule,
- 80-100 mg xanthan gum,
- 30-50 mg tara gum, and
- 5-20 ml of water.

The composition of the present invention may be prepared using methods known in the art.

However, a preparation process has been developed which ensures the properties of the resulting composition remain optimal over time, in terms of both activity and stability. The present invention therefore also concerns a process for the preparation of the composition of the invention, comprising the steps of:
i) dissolving said hyaluronic acid or salt thereof in water,
ii) adding said natural molecule under stirring,
iii) adding said at least one salt of alginic acid under stirring,
iv) adding said at least one thickening agent under stirring, and
v) stirring for at least 60 minutes at about 30-50°C at a pH of 6-8.

Preferably, during step i), the hyaluronic acid or salt thereof is stirred for at least five minutes at a temperature no higher than 40°C. More preferably, step i) is carried out under vacuum.

Preferably, step ii) is carried out under vacuum.

Preferably, step iii) is carried out under vacuum.

Preferably, step iv) is carried out under vacuum.

In another aspect, the present invention relates to the use of the composition described above in the treatment of gastroesophageal reflux and laryngopharyngeal reflux. As already stated, indeed, the composition of the invention offers advantageous reparative and regenerative properties with respect to the mucosa, with consequent rapid relief from symptoms, featuring, at the same time, significant rheological characteristics both in terms of transit and in terms of mucoadhesive properties of the components thereof, with a direct causality between contact time and efficacy.

The composition of the invention therefore benefits from the association of the salt of alginic acid and the active component, i.e. from the mucoadhesive, regenerating and filmogenic characteristics, as well as from the important conveyance of water, hyaluronic acid or the salt thereof, strengthened by the presence of the natural molecule, in synergy with the salt of alginic acid.

The efficacy of the association is enhanced and optimised by the contemporaneous presence of at least one thickening agent, which has proper affinity with the proteins of the gastroesophageal mucosa. This allows the composition of the invention to form a film on the surface of the mucosa, thus increasing contact times and therefore supporting the protective and regenerative effect. Therefore, a synergy was observed between the action of the active component and the action of the salt of alginic acid, since the latter shields the gastric reflux, while also offering the advantage of longer contact time between the active component and the mucosa to be regenerated.

In this sense, the composition of the invention is not only efficacious in the treatment of gastroesophageal reflux, but also of disorders associated therewith which also affect the otorhinolaryngological, pharyngeal, and pneumological systems, including the oropharynx, nasopharynx, the middle ear, the nasal/sinusoidal cavities, and even the conjunctival mucosa. Etiological agents include hydrochloric acid and (more predominantly) pepsin, which - since located in an environment other than the gastroesophageal environment - cause significant changes in the mucosa of the upper airways, which are responsible for symptoms such as: pyrosis, epigastralgia, hiccups, acid regurgitation, retrosternal pain, dysphonia, foreign body sensation, excess mucus in the pharynx, coughing, oropharyngeal burning, laryngeal spasms, odynophagia, halitosis, dyspnoea, rhinorrhea, nasal obstruction, ear fullness, through to conjunctival burning, and lacrimation.

It follows that the composition of the invention has a biological polyvalence which ranges from modulation of the inflammatory process both directly (by inhibiting GER) and indirectly, i.e. by acting - in association with the properties of the hyaluronic acid or salt thereof - to modulation of the mucosa re-epithelisation process, through the action of the natural molecule and the hyaluronic acid or salt thereof.

Furthermore, advantageously, the composition of the invention does not interfere with the action of antisecretory drugs (known as 'proton pump inhibitors' or PPIs) and can therefore be administered contemporaneously therewith.

Preferably, the composition of the invention is administered orally.

More preferably, the composition of the invention is provided in a single-dose form of 1-50 ml, more preferably 5-30 ml, and even more preferably 7-20 ml.

It should be understood that all the possible combinations of the preferred aspects of the composition components, the unit doses, the preparation, and the uses of said composition are described herein and therefore are also preferred.

It is also to be understood that all aspects identified as favourable and advantageous for the composition and the components thereof, should be deemed equally preferable and advantageous also for the preparation and the uses of said composition.

Below are working examples of the present invention provided for illustrative purposes.

### EXAMPLES

### Example 1.

The following composition was prepared:

| | % w/w | mg |
|---|---|---|
| Magnesium alginate | 0.25 | 37.5 |
| Sodium hyaluronate | 0.10 | 15.0 |
| Hydrolysed keratin | 0.10 | 15.0 |
| Xanthan gum | 0.60 | 90.0 |
| Tara gum | 0.25 | 37.5 |
| Purified water | balance to 100% | |

The composition stated above was marked with methylene blue and orally administered to patients. The methylene blue allows to follow the movement of the composition along the gastroesophageal mucosa by means of endoscopic viewing and, for two minutes, the presence of the composition was observed at the upper third, middle and lower level of the oesophagus, in addition to the gastric cavity.

This confirmed the high mucoadhesivity of the composition along the walls of the areas to be treated.

### Example 2.

The following composition was prepared:

| | % w/w | mg |
|---|---|---|
| Magnesium alginate | 0.10 | 15.0 |
| Sodium hyaluronate | 0.15 | 22.5 |
| Hydrolysed keratin | 0.05 | 7.5 |
| Locust bean gum seeds | 0.50 | 75.0 |
| Gum arabic | 0.30 | 45.0 |
| Purified water | balance to 100% | |

The composition stated above was marked with methylene blue and orally administered to patients. The methylene blue allows to follow the movement of the composition along the gastroesophageal mucosa by means of endoscopic viewing and, for two minutes, the presence of the composition was observed at the upper third, middle and lower level of the oesophagus, in addition to the gastric cavity.

This confirmed the high mucoadhesivity of the composition along the walls of the areas to be treated.

### Example 3.

The following composition was prepared:

| | % w/w | mg |
|---|---|---|
| Magnesium alginate | 0.25 | 50.0 |
| Potassium hyaluronate | 0.05 | 7.5 |
| Hydrolysed keratin | 0.15 | 22.5 |
| Guar gum | 0.80 | 120.0 |
| Sodium alginate | 0.40 | 60.0 |
| Purified water | balance to 100% | |

The composition stated above was marked with methylene blue and orally administered to patients. The methylene blue allows to follow the movement of the composition along the gastroesophageal mucosa by means of endoscopic viewing and, for two minutes, the presence of the composition was observed at the upper third, middle and lower level of the oesophagus, in addition to the gastric cavity.

This confirmed the high mucoadhesivity of the composition along the walls of the areas to be treated.

### Example 4.

The following composition was prepared:

| | % w/w | mg |
|---|---|---|
| Magnesium alginate | 0.25 | 37.5 |
| Potassium hyaluronate | 0.05 | 7.5 |
| Collagen | 0.15 | 22.5 |
| Fibronectin | 0.80 | 120.0 |
| Carrageenan | 0.40 | 60.0 |
| Purified water | balance to 100% | |

The composition stated above was marked with methylene blue and orally administered to patients. The methylene blue allows to follow the movement of the composition along the gastroesophageal mucosa by means of endoscopic viewing and, for two minutes, the presence of the composition was observed at the upper third, middle and lower level of the oesophagus, in addition to the gastric cavity.

This confirmed the high mucoadhesivity of the composition along the walls of the areas to be treated.

### Example 5.

The following composition was prepared:

| | % w/w | mg |
|---|---|---|
| Magnesium alginate | 0.40 | 60.0 |
| Sodium hyaluronate | 0.10 | 15.0 |
| Integrin | 0.10 | 15.0 |
| Laminin | 0.60 | 90.0 |
| Heparan sulphate | 0.25 | 37.5 |
| Purified water | balance to 100% | |

The composition stated above was marked with methylene blue and orally administered to patients. The methylene blue allows to follow the movement of the composition along the gastroesophageal mucosa by means of endoscopic viewing and, for two minutes, the presence of the composition was observed at the upper third, middle and lower level of the oesophagus, in addition to the gastric cavity.

This confirmed the high mucoadhesivity of the composition along the walls of the areas to be treated.

### Example 6.

The following composition was prepared:

| | % w/w | mg |
|---|---|---|
| Magnesium alginate | 0.25 | 37.5 |
| Sodium hyaluronate | 0.10 | 15.0 |
| Hydrolysed keratin | 0.10 | 15.0 |
| Xanthan gum | 0.60 | 90.0 |
| Tara gum | 0.25 | 37.5 |
| Potassium sorbate | 0.10 | 15.0 |
| Sodium benzoate | 0.10 | 15.0 |
| ε-polylysine | 0.05 | 7.5 |
| Sucralose | 0.024 | 3.6 |
| Mint flavouring | 0.20 | 30.0 |
| Purified water | balance to 100% | |

The composition stated above was marked with methylene blue and orally administered to patients. The methylene blue allows to follow the movement of the composition along the gastroesophageal mucosa by means of endoscopic viewing and, for two minutes, the presence of the composition was observed at the upper third, middle and lower level of the oesophagus, in addition to the gastric cavity.

This confirmed the high mucoadhesivity of the composition along the walls of the areas to be treated.

## Claims

1. A composition comprising:
i) at least one salt of alginic acid, and
ii) an active component comprising:
- hyaluronic acid or a salt thereof, said salt being selected from sodium hyaluronate, potassium hyaluronate, calcium hyaluronate, and mixtures thereof,
- a natural molecule selected from glycosaminoglycan, glycoprotein, keratin, collagen, and mixtures thereof, and
- at least one thickening agent,
wherein said at least one salt of alginic acid and active component are in a weight ratio of 1:1 to 1:15.

2. The composition of claim 1, wherein said composition further comprises water and has pH of 6-8.

3. The composition of claim 1 or 2, wherein said at least one salt of alginic acid and active component are in a weight ratio of 1:2 to 1:10.

4. The composition of any one of claims 1-3, wherein said at least one salt of alginic acid is magnesium alginate.

5. The composition of any one of claims 1-4, wherein said at least one thickening agent and hyaluronic acid or a salt thereof are in a weight ratio of 4:1 to 12:1, and said at least one thickening agent and natural molecule are in a weight ratio of 4:1 to 12:1.

6. The composition of any one of claims 1-5, wherein said natural molecule is keratin.

7. The composition of any one of claims 1-6, wherein said at least one thickening agent is a mixture of xanthan gum and tara gum.

8. The composition of any one of claims 1-7, in the form of a unit dose comprising:
- up to 200 mg of at least one salt of alginic acid,
- up to 3000 mg of active component, and
- up to 100 ml of water.

9. The composition of any one of claims 1-8, in the form of a unit dose comprising:
- 20-50 mg of at least one salt of alginic acid,
- 10-20 mg of hyaluronic acid or salt thereof,
- 10-20 mg of natural molecule,
- 100-200 mg of a mixture of xanthan gum and tara gum, and
- 5-20 ml of water.

10. The composition of any one of claims 1-9 for use in the treatment of gastroesophageal reflux and laryngopharyngeal reflux.
